(19) **Europäisches Patentamt / European Patent Office / Office européen des brevets**

(11) **EP 4 450 245 A1**

(12) **EUROPEAN PATENT APPLICATION**

(43) Date of publication:
**23.10.2024  Bulletin 2024/43**

(21) Application number: **23168822.7**

(22) Date of filing: **19.04.2023**

(51) International Patent Classification (IPC):
**B28C 7/02** *(2006.01)*    **B28C 7/16** *(2006.01)*
**E04G 21/02** *(2006.01)*    **F04B 15/02** *(2006.01)*
**G01N 33/38** *(2006.01)*

(52) Cooperative Patent Classification (CPC):
**B28C 7/026; B28C 7/163; F04B 1/02;
F04B 9/1172; F04B 15/02; F04B 17/03;
F04B 49/06; F04B 49/08; F04B 49/10;
G01N 33/383; F04B 2203/0201**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Sostaric, Joseph
Danville, CA 94506 (US)**

(72) Inventor: **Sostaric, Joseph
Danville, CA 94506 (US)**

(74) Representative: **Boult Wade Tennant LLP
Salisbury Square House
8 Salisbury Square
London EC4Y 8AP (GB)**

(54) **SYSTEM AND METHOD FOR MEASURING SLUMP OF CONCRETE MIX IN A PUMP HOPPER**

(57)    A device and method for measuring slump of concrete in a hopper (20) feeding a concrete pump. A slump sensor generates a signal from either an ammeter (55) to measure current from a power source (51) feeding an electric motor (41) being configured to rotate an agitator shaft (31) or a pressure gauge configured to measure hydraulic pressure in a hydraulic drive hose passing between a hydraulic pump and a hydraulic motor configured to rotate the agitator shaft. A controlling computer (61) includes a memory in which an operating range may reside, the operating range being a range from a lower slump signal value corresponding to a lower limit of slump value to an upper slump signal value. The controlling computer (61) generates an alert signal when the slump sensor signal falls outside of the operating range. In one embodiment, the controlling computer stops the concrete pump (10) when the alert signal is detected.

**FIG.2**

## Description

## PRIORITY CLAIM

This application is a utility application claims priority from and entirely incorporates the Provisional Application filed as Serial No. 63/252,002 on October 4, 2021, entitled "System and Method for Measuring Slump of Concrete Mix in a Pump Hopper" by Joseph Sostaric.

## FIELD OF THE INVENTION

This disclosure relates to the field of hydraulic concrete pumping and more specifically to the method for measuring and monitoring the slump flow of concrete as it passes through a hydraulic concrete pump as well as the use of a continuous instantaneous measurement of slump to prevent introduction of air into pumping lines.

## BACKGROUND OF THE INVENTION

Setting of concrete represents the transition phase between a fluid and a rigid state. This period starts when concrete loses its plasticity, becoming unworkable, and it is complete when it possesses enough strength to support loads with acceptable and stable deformation. At the end of the setting period, concrete continuously gains strength with time in the subsequent hardening period. Rheological properties of fresh concrete vary steadily within the initial and final setting times with consequent decrease in workability, as well as increase of energy consumption at the subsequent consolidation.

The properties of fresh concrete are extremely important. Consistency and workability of fresh concrete are significant criteria for the concrete mix design proportioning and important properties affecting the placing of fresh concrete on site and the later performance of the hardened state of concrete.

Workability represents diverse characteristics of freshly mixed concrete that are difficult to measure quantitatively. Workability involves certain characteristics of fresh concrete such as cohesiveness and consistency.

Cohesiveness (stability) is a measure of the compactability and finishability of fresh concrete. Compacting factor test is used to evaluate the compactability characteristics of a concrete mixture. Consistency, which is the relative mobility or ability of freshly mixed concrete to flow, is a measure of the wetness of the fresh concrete mix. It is evaluated in terms of slump, and it is the most widely used test for concrete at construction site. The required workability depends on the type of construction, placement method, consolidation method, shape of formwork and structural design.

The 'slump' of concrete refers to the consistency of fresh concrete before it sets - the higher the slump, the more fluid the concrete is. Simply put, the higher the slump, the wetter the mix. Slump is a test to measure the plasticity (wetness) of concrete when it is placed. The distance the concrete slumps (sags or settles down) is the slump. Wetter concrete slumps further. A normal slump range (U.S.) is 2 to 4 inches, which means a slump cone 12 inches tall is filled with the fresh concrete, it is rodded to consolidate it, then the cone is lifted off the 'mound' of concrete. The slump measurement process is defined by ASTM C143 - Standard Test Method for Slump of Hydraulic-Cement Concrete.

The conventional procedure by which to determine concrete slump is generally performed using a standardized Slump Cone Mold-The mold is 12-in. in height and is also known as an Abrams cone. Securing a clean Abrams cone to its corresponding base plate serves as a mold to form a uniform cone of concrete mix. Thus, the user will feed a concrete sample into the mouth of the cone. To assist in filling the cone and assuring that voids or air bubbles will not prevent the mix from fully filling the cone for accurate measurement, the user will use a steel tamping rod-Generally such a tamping rod is a 5/8-in. diameter, smooth-surfaced, 24-in. long rod with hemispherical (rounded) tips. Once fully filled and inverted with the base plate in place, the Abrams cone is removed to present a free-standing truncated cone of concrete mix which height is then measured to present a value.

More specifically, the process of standardized measurement, performed to conform with the method expressed in ASTM C172, is initiated as the cone is filled within five minutes of collecting and remixing the sample. The cone is filled and rodded in thirds, rodding the mixture 25 times evenly across the sample to assure homogeneity of the mix after each third is introduced into the cone. Once properly filled and suitably struck off to level the concrete mix to the throat of the mold, the mold is inverted on its base and then removed. In this manner the height of the free-standing truncated cone of concrete mix is measured against the height of the inverted mold. The difference in height is the measure of the slump of the concrete. Slump informs a number of processes including that of pumping concrete.

Pumping concrete through metal pipelines by piston pumps was introduced in the United States in Milwaukee in 1933. This concrete pump used mechanical linkages to operate the pump and usually pumped through pipelines 6 in. or larger in diameter. Pumping may be used for most concrete construction but is especially useful where space for construction equipment is limited. Concrete pumping frees hoists and cranes to deliver the other materials of construction concurrently with concrete placing. Also, other crafts can work unhampered by concrete operations rendering as free the ground area that would be occupied by a simple pour to form.

When forming concrete is required, a mixing truck first mixes concrete within its rotating drum. If the mix is to be pumped, the mix truck then pours its liquid concrete mix into a hopper of a concrete pump. The hopper of the mix truck preserves plasticity of the mix as an agitator shaft having agitating paddles extending radially from the agitator shaft rotating in the mix so the mix will not solidify. From hopper, the concrete pump sucks the liquid concrete from the hopper, through a valve system and a discharge pipe into a series of several pipes, and out of the pipes into the area where it needs to be laid down, sometimes requiring the use of auxiliary hoses.

To assure continued plasticity of the concrete, according to conventional means, an operator will measure the slump of the concrete in the drum of the ready-mix concrete truck. However, such measurements occur prior to the transfer from the drum to a concrete pump hopper. To date, there has been no conventional method to measure the concrete's workability once it has been discharged into the hopper feeding the concrete pump.

In practice, if the concrete mix in the truck has a suitable slump, the operator will pour that mix from the mixing truck into the hopper. Once the concrete mix is deposited into the hopper of the pump, however, the slump can change even as the mix resides in the hopper. When working on a big project with several loads of concrete, it would be desirable to perform a slump test; knowing the slump of the mix can help to ensure that each pour of a load of concrete is uniform and, when poured, homogenous with the earlier and later pours into the form. Homogeneity in the mix assures strength of the resulting structure as the concrete sets uniformly within the form.

In conventional pumping of concrete, the concrete is formulated to have a limited amount of time in which it can be pumped. Hardening is the result of cementitious material found in concrete which in hydration generates heat in an exothermic reaction. As the cement hydrates the concrete will lose slump and change to a hardened state. Conventionally, a known target interval is specified, thereby to allow homogeneity in the several pours of concrete to be pumped into such form that serves as mold for the finished casting. Such an interval is generally selected to allow suitable filling of the form while assuring that hydration can begin at an advantageous rate and time. Pumping allows such a rapid series of pours to properly meet the specified interval.

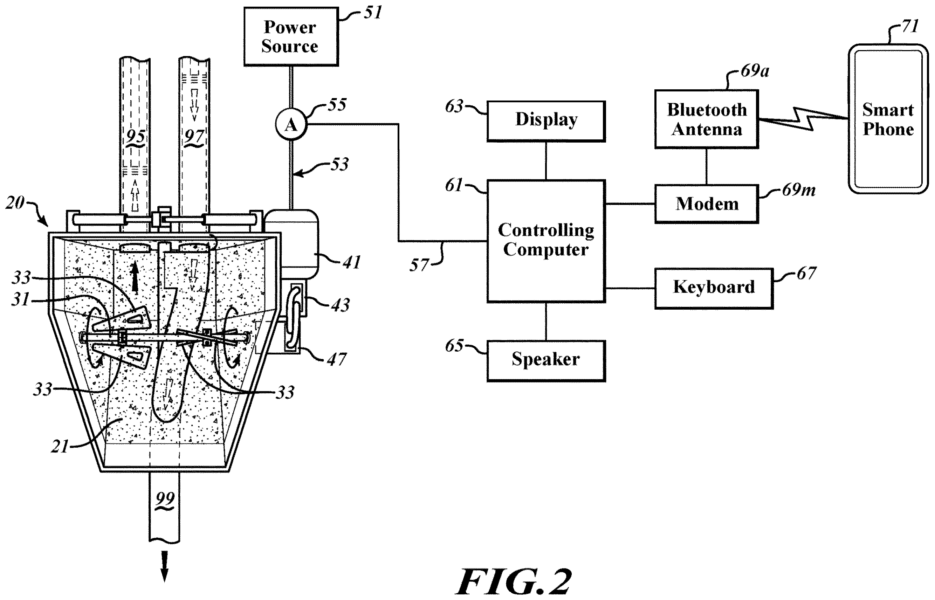

Generally, construction specifications typically call for the concrete to be placed within 90 minutes from the time it is initially batched. Such a specification points to a limited shelf life of concrete while it remains in a plastic state. This specified interval not only assures a stronger final structure but also preserves the pumping equipment. When concrete begins to harden, it becomes stiffer and less workable where the pressure required to pump the concrete will increase dramatically. Where the interval is exceeded, the concrete in the pump can set up and harden. Hardened concrete will render pipes unusable requiring complete replacement of all the pipe. Further, other areas of the pump 10, such as the hopper 20 and the cylinders 95, 97 (Figure 2), will need to be jack-hammered to clean hardened concrete from interior surfaces.

Another issue that the pumping of concrete presents is a danger that occurs when the concrete pump draws air into the cylinders 95, 97 which is used for motivating the concrete through the pipes. Such air is then compressed by the pump and might be sealed in the discharge pipe 99 by later ingestion of concrete into the pump. Most often air in pumping lines occurs when the pump draws down all the concrete mix in the hopper but continues to draw. When the pump cylinder 95, 97 inlet ports are exposed to the ambient air by the receding concrete mix, it draws that ambient air into the lines. By way of analogy, it is very like a child drawing a soft drink up a straw. The slurping sound that occurs when the soft drink does not cover the lower end of the straw and the child sucks up the ambient air.

Concrete pumping relies upon the incompressible nature of a plastic concrete mix as it is pressed into the pipe. As in any pipe system, the more highly placed concrete mix within the pipes is supported by the mix below it; any air between the two masses of concrete mix will be compressed by gravity acting on the upper mass of mix. The pressure of air in the line or pipe is compressed to a pressure sufficient to support the upper mass of concrete mix. Where air is in the pipes and is compressed sufficiently to support the upper mass of concrete, that extremely high state of compression can set up the hose for filling forms as the nozzle of a jet. When the air escapes as that air bubble reaches the nozzle and the forces upon escaping will whip that hose wildly.

From a safety perspective, the release of that highly compressed air is extremely dangerous. The escaping jet of air can drive the nozzle at extremely high speeds in an unpredictable manner. As concrete pumps are often operated remotely and where the operator stands near the point of placement manually guiding the hose, a wildly oscillating whipping nozzle can injure or kill an operator. The best way to prevent the introduction of air into the cylinders is to keep the pump cylinder 95, 97 ports immersed in the concrete mix, preventing air from reaching the cylinders 95, 97.

One way to maintain a suitable level of concrete mix in the hopper is to have an operator to slow or to shut off the pump when the hopper is near its lower extreme of operation prior to exposing the pump cylinder 95, 97 ports. As the hopper empties, the vanes of the auger or ends of paddles of the agitator are exposed to the air before air is entrained in the concrete mix. Thus, an alert operator can interrupt the operation of the pump any time tips of paddles of the agitator are exposed and visible. But, human operators are not infallible on that point. As more of the paddles are exposed, the agitator rotates with less effort within the hopper.

What is needed in the art is mechanism to measure slump of a concrete mix as it resides in a hopper to assure a suitable flow of concrete having an appropriate slump and does so to the exclusion of air from the concrete pumping pipes.

## SUMMARY OF THE INVENTION

The present invention continuously and instantaneously measures and monitors the concrete slump as the mix is drawn into the concrete pump. Knowing the slump of concrete mix in the hopper can, as described herein, provide a more accurate assessment of the state of the concrete that has been discharged to its final point of placement.

A device and method for measuring slump of concrete in a hopper feeding a concrete pump. A slump sensor generates a signal from either an ammeter to measure current from a power source feeding an electric motor being configured to rotate an agitator shaft or a pressure gauge configured to measure hydraulic pressure in a hydraulic drive hose passing between a hydraulic pump and a hydraulic motor configured to rotate the agitator shaft. A controlling computer includes a memory in which an operating range may reside. The operating range is defined as a range from a lower slump signal value corresponding to a lower limit of slump value to an upper slump signal value. The controlling computer generates an alert signal when the slump sensor signal falls outside of the operating range. In one embodiment, the controlling computer stops the concrete pump when the alert signal is detected.

Any conventional concrete pump uses an electric motor turning the shaft of a hydraulic pump generating pressure to motivate rotation of a hydraulic motor to pump the concrete. Because it is the current energizing the electric motor that rotates the shaft of the pump, rotation of the motor's shaft drives the pump and it is the pump drives hydraulic fluid that, in turn, drives the rotation of the agitating shaft. In this manner, current expended by the motor is proportionate to the work necessary to agitate the concrete mix. Thus, the amperage is proportionate to the work expended to turn the paddles. Either that current, or in an alternative embodiment, a hydraulic pressure sensed in hydraulic lines as measured with a hydraulic pressure sensor yield an indication of work expended. In the case of an electric motor, the current required to turn the paddles can be measured by an ammeter and, thus, known. Once known, the either electric current or hydraulic pressure can be measured and can be recorded with a time stamp and either current or hydraulic pressure can be used as a surrogate to the standard ASTM C172 procedure for measurement of the concrete consistency or slump.

## BRIEF DESCRIPTION OF THE DRAWINGS

Preferred and alternative examples of the present invention are described in detail below with reference to the following drawings:

Figure 1 depicts a conventional concrete pumping device, in this nonlimiting instance, the pumping device is configured as a trailer; and

Figure 2 depicts an agitator motor with the hydraulic lines that power the rotation of the exemplary hydraulic agitator along with the amperage sensor feeding a computer.

## DETAILED DESCRIPTION OF THE PREFERRED EMBODIMENT

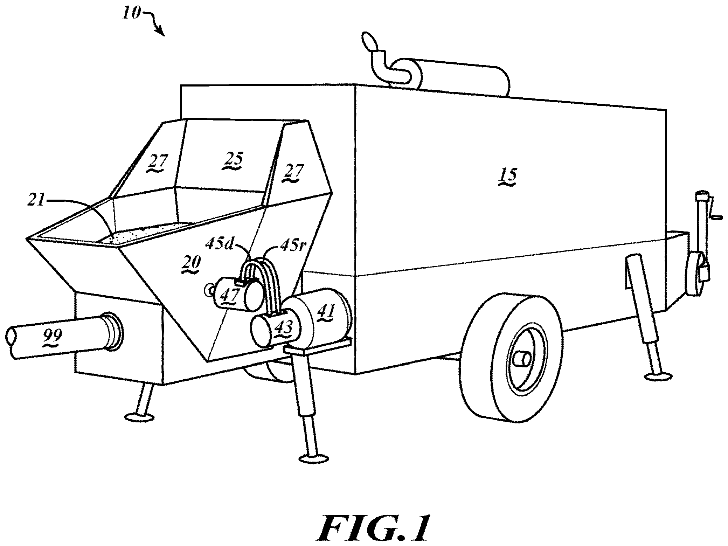

Referring to Figure 1, the most common concrete pumps 10 consist of a receiving hopper 20, two concrete pumping cylinders 95, 97 (Figure 2), and an S-shaped discharge tube 99 is moved to alternately direct the flow of concrete into the pumping cylinders and from them to the pipeline. The exemplary embodiment shown in Figure 1 includes a shroud 15 that covers the pump mechanism (not shown) which moving mechanism is thereby enclosed for the safety of the operators and shielding of the moving mechanism including two concrete pumping cylinders 95, 97 (Figure 2), and a valving system (not shown). Given that the illustration set out as Figure 1 includes the shroud 15, the specific mechanism of the pump 10 is not shown nor is it necessary to understand the invention in that all that is important occurs in the hopper 20.

The hopper 20 has, in some embodiments, a mesh grate (not shown for purposes of illustration) strains the concrete 21 as it falls through, the mesh grate prevents any large rocks or chunks from plugging the pump or the pump truck hoses. As the invention does not rely upon the mesh grate or its presence, in the illustration, it has been omitted for clarity. The hopper 20 also has an agitating auger comprising an agitator shaft 31 having a plurality of agitator paddles 33 which extend radially from the agitator shaft 31 that together churn the concrete keeping it liquid and flowable. Because the grate has been omitted from the illustration, in the diagram, concrete 21 is visible in the hopper 20. A hopper backsplash 25 and two hopper sidesplashes 27, are illustrated and, in filling the hopper 20 are used to direct concrete into the hopper 20. These too, are not relevant to the operation of the invention but are used in conventional pumping of concrete.

In conventional pumping and in the inventive use herein, a ready-mix truck will deposit concrete 21 into the hopper 20. Though the hopper 20 may vary in shape from pump 10 to pump 10, its capacity, and the nature of any reinforcement of the hopper 20 designed to withstand forces exerted by heavy concrete 21 do not affect the operation of the inventive device. All hoppers 20 are configured to assure a steady feed of concrete 21 with agitator paddles 33 to maintain plasticity of mix within the hopper 20. As indicated earlier in the background section herein, too little hopper 20 capacity can have serious consequences if the operator is not mindful of the level therein.

As set out in the background, it takes only a few seconds for the flow of concrete 21 into the pump 10 from the hopper 20 to draw down the level of concrete 21 within the hopper and thereby to allow the pump to cavitate, filling the pipes with compressed air between masses of concrete. The hopper 20 is not a silo holding concrete 21; it is a funnel through which the concrete 21 flows. From the bottom of the hopper 20, the concrete 21 is drawn into one of two cylinders (not shown) comprising the pump. The concrete 21 is then forced out of the cylinder, by way of a valve, into the hose where it eventually finds its way to the far end for placement into forms, block walls, or slabs. As stated above, compressed air in the pipes and hoses poses an extreme hazard to crew safety.

Referring to Figure 1, a conventional concrete pump 10 is driven by a pressurized flow of hydraulic fluid, also known as hydraulic fluid, most likely fed by any of several rotary machines such as a gasoline engine, diesel engine, or electric motor driving an axial piston pump. The movement of concrete by a conventional concrete pump 10 relies upon a fully hydraulic, twin-cylinder, reciprocating design. The conventional concrete pump 10 generally comprises two double-acting hydraulic cylinders (not shown) to motivate two pistons (not shown) reciprocating in the two hydraulic cylinders. During operation, a control valve box alternately pressurizes the rod-side of one of the hydraulic pistons to cause movement within the hydraulic cylinder. Each of the two hydraulic pistons move back and forth (i.e., reciprocates) to similarly move a material piston connected by a connecting rod to the corresponding hydraulic piston. Much like the legs of a pedaling bicyclist, the pistons are 180 degrees out of synchronization, i.e. when one hydraulic piston reaches the end of its stroke the other reaches the opposite end. A rocking valve selectively exposes each material cylinder 95, 97 to a flow from a concrete hopper or flow into a discharge tube 99 (in this exemplary drawing, an "S"-shaped discharge tube) 99. In this manner, the two material pistons pump concrete from the hopper and into the discharge tube 99.

As indicated, in a conventional concrete pump 10, an electric motor 41 rotates the shaft of a hydraulic pump 43. In turn, the hydraulic pump 43 presses hydraulic fluid into a hydraulic drive hose 45d at high pressure. After the pressure of the hydraulic fluid in the hydraulic drive hose 45d is expended in rotating the hydraulic motor 47 shaft, the fluid returns to the hydraulic pump 43 though a hydraulic return hose 45r. A hydraulic motor 47 is a mechanical actuator that converts hydraulic pressure and flow into torque and angular displacement (rotation). Pressurized hydraulic fluid received from the hydraulic pump 43 turns the hydraulic motor 47 output shaft by pushing on the gears, pistons, or vanes of the hydraulic motor 47. The hydraulic pump 43 rotates the agitator shaft 31 and the agitator paddles 33 which extend radially from the agitator shaft 31, thereby agitating the concrete 21 in the hopper 20.

In any electrical system, voltage is the force required to move electrons. Current is the rate of the flow of charge per second or electrical current through a material to which a specific voltage is applied. By taking the voltage and multiplying it by the associated current, the power can be determined.

$$P = V * I$$

where power (P) is in watts, voltage (V) is in volts and current (I) is in amperes

A watt (W) is a unit of power defined as one Joule per second. For a DC source the calculation is simply the voltage times the current: W = V x A. However, determining the power in watts for an AC source must include the power factor (PF), so W = V x A x PF for AC systems. In either system, current is proportionate to work performed.

Since power is voltage times current (P= V*I), power is highest when the voltage and current are lined up together so that the peaks and zero points on the voltage and current waveforms occur at the same time. In an electric motor, the mechanical power is defined as the speed times the torque. Mechanical power is typically defined as kilowatts (kW) or horsepower (hp) with one watt equaling one joule per second or one Newton-Meter per second.

The current through a motor 41 is proportional to the torque produced by the motor 41. Normally the back electromotive force created by the speed of the motor 41 opposes the voltage being applied and limits the amount of current the motor 41 draws and thus it's torque. As more and more load is applied to the motor 41, the motor 41 slows reducing its back EMF allowing more current to flow, producing more torque. Thus, by measuring the current draw of an electric motor 41 under otherwise known conditions, the operator can relate the current draw at the electric motor 41 driving the hydraulic pump 43, one might measure the workability or slump of concrete 21 being agitated as proportionate to the work performed.

Referring to Figure 2, the present invention monitors current drawn at a known rotational speed to determine the slump of concrete 21 within the hopper 20. In an alternate embodiment, a pressure sensor in the "high" side of the hydraulic hose 45d can be used to similarly measure workability of the concrete 21 mix in the hopper 20. In these two alternate embodiments, either measurement, i.e. that of drawn amperage or that of high side pressure in the hydraulic driving hose 45d, acts as a measure the resistance of concrete 21 to movement when driven by the agitator paddles 33. Thus, either of drawn amperage or high side pressure in the hydraulic driving hose 45d will be proportionate to the slump of concrete 21 in the hopper 20 and related to either by a selected constant coefficient.

Still further, the torque asserted against the agitator paddles 33 is a function of the plasticity of concrete 21 against which the agitator paddles 33 press when rotating. So, for example, when the agitator paddles 33 press against ambient air, as opposed to as against concrete 21, the agitator shaft 31 will rotate relatively freely when compared to the movement of the paddles 33 when agitating concrete 21. Either of the ammeter 55 in the original embodiment or the pressure gauge (not shown) in the second embodiment, detect the lack of a significant resistance in rotating the agitator shaft 31, the agitator paddles 33 press only against ambient air, the agitator paddles 33 perform less work to turn against the lowered level of concrete than the same agitator paddles 33 might require in a fully loaded hopper 20. Thus, when the amperage measured at the ammeter 55 falls below a selected level, the inventive pumping assembly detects that the concrete hopper 20 is empty of concrete (or at least lowered) due to the lack of resistance as the agitator paddles 33 encounter as they pass through the air. When an empty hopper 20 event is detected, that is when amperage at a known rotational speed falls below a threshold for safe operation, the controlling computer 61 can immediately either alert the operator by any of sounding a warning tone on a speaker 65, flashing a warning on a display 63, or by sending suitable signals to a Bluetooth modem 69m for transmission on a Bluetooth antenna 69a to an application running on a smart phone 71 or in a different optional embodiment, to stop the pump 10 entirely.

In this way, the inventive controlling computer 61 assures that the level of concrete 21 in the hopper 20 will always be sufficient to prevent ingestion of air into the pump cylinders 95, 97 as the pump 10 operates. By preventing the ingestion of air into the pump 10, the controlling computer 61 prevents a catastrophic "hose whipping" because the pump 10, in some inventive embodiments, will either slow or stop the drawing of concrete mix 21 through the hopper 20 until more concrete mix can be added to the hopper 20, thereby keeping the cylinders 95, 97 submerged.

In computer science, a lookup table (LUT) is an array that replaces runtime computation with a simpler array indexing operation. The process is termed as "direct addressing" and LUTs differ from hash tables in a way that, to retrieve a value "v" with a key "k", a hash table would store the value "v" in the slot "h(k)" where "h" is a hash function i.e. "k" is used to compute the slot, while in the case of LUT, the value "v" is stored in slot "k," thus directly addressable. The savings in processing time can be significant, because retrieving a value from memory is often faster than carrying out an "expensive" computation or input/output operation. The tables may be precalculated and stored in static program storage, calculated (or "pre-fetched") as part of a program's initialization phase (memorization), or even stored in hardware in application-specific platforms. Lookup tables are also used extensively to validate input values by matching against a list of valid (or invalid) items in an array and, in some programming languages, may include pointer functions (or offsets to labels) to process the matching input. FPGAs also make extensive use of reconfigurable, hardware-implemented, lookup tables to provide programmable hardware functionality.

In embodiments of the invention, the accessing of a lookup table containing values in a "mapping" of amperages, rotational speeds, ambient temperature, temperature of the concrete mix 21, ambient humidity, and the, thus-measured, slump, enables the controlling computer 61 to assure only the concrete mix 21 will reach the pump 10.

Further, monitoring the process closely allows a controller to develop a "map" indicating states of hydration of concrete 21 in the hopper 20 while pumping, to optimize the state of concrete as it is pumped, or, also, to alert an operator when a quality of cement mix, the slump, falls outside of a recommended range. By controlling the pumping based upon a sensed state of the concrete 21, the controlling computer 61 additionally, preserves the hopper 20 and pipes such that they can be suitably emptied before the concrete can permanent ruin component parts of the pumping assembly 10.

One of these optional ambient sensors bears further explanation. The concrete temperature proves to be important as it bears on the instantaneous measurement of the slump of the concrete. The concrete temperature sensor or thermometer described in embodiments of the invention will allow the system to monitor the temperature of the concrete as it flows through the concrete pump. At present, no pump manufacturers are measuring, monitoring or recording the concrete temperatures. Because the hydration process is an exothermic reaction, monitoring heat gain (temperature rise) in conjunction with slump loss data will forewarn the operator that the concrete in the pump has reached the point where it may no longer be useable and should therefore be discarded and flushed from the pump. This is a distinct and useful warning to the operator. Elevated temperatures occur when placing concrete that has already begun its initial set; using concrete that has advanced through this initial set can cause structural or aesthetic deficiencies. Placing such hardened mix results in the finally cured concrete exhibiting lower compressive strength, excessive cracking, improper finish or other consolidation issues. Simply put, defects can occur when placing concrete including partially hydrated cement.

Likewise, even the mix of the concrete can be measured by embodiments of the invention. It is known that the addition of too much water to the concrete mix will give the mix an excessive slump which, in the resulting cured concrete will cause either or both of excessive cracking or reduction in compressive strength. Thus, the slump data compiled from the pump can then further serve Quality Assurance or Quality Control functions by validating workability characteristics during placement. Where an embedded controlling computer 61 using either the amperage-draw or the sensed hydraulic pressure motivating the agitator or auger falls outside of certain known parameters, the concrete 21 can be diverted from the forms in favor of a more favorable mix. It is also possible to send this information to the controlling computer 61 on the pump 10 so that an adjustment to the flow of concrete 21 can be made automatically which will avoid placing substandard cement 21.

In a "learning" mode of the controlling computer 61, measured slump in the hopper 21 can be matched to values of either of drawn amperage or high side pressure in the hydraulic driving hose 45d to determine a suitable coefficient with which to multiply such value to measured slump. The monitoring of either of the pressure in the hydraulic drive line 45d or the monitoring of amperage necessary to rotate the agitating shaft 31 with its agitator paddles 33 as sensed is "learned" by an artificially intelligent regimen to work up and populate a "lookup table". "Teaching" the artificially intelligent controller and thereby populating the lookup table is an iterative process of recording and storing all corresponding measurements and using learned values for predictive analytics. By retrieving these learned values when in an "operating" mode the controlling computer 61, will serve to keep the pump 10 in an optimal pumping mode relative to the state of the concrete mix 21 in the hopper 20. In another embodiment, rather than learning the values to populate a lookup table, the controlling computer 21 relies upon a preprogrammed "lookup table" whereby the controlling computer 61 can suitably adjust the rate by which concrete 21 is discharged from the pump 10.

In various embodiments, the controlling computer 61 of the invention relies upon an operating range based upon the measurements of either current draw by the motor 41 as measured at an ammeter 55 or, in the alternate embodiment, pressure measured by a pressure gauge (not shown) measuring pressure in the driven hydraulic hose 45d. Based upon the received signals from either of the ammeter 55 or the pressure gauge (not shown), the operating range might be varied as a function of additional optional variables such as ambient humidity, ambient temperature, temperature of the concrete 21, though none of these optional variables are necessary for the operation of the invention. In combination, the controlling computer 61 can determine the interval a concrete mix will remain suitably plastic for pumping. In such embodiments, not only can the invention judge the instantaneous workability of the concrete mix but can additionally predict the expected workability of the concrete mix 21 during the process of pumping through a hydraulic pump 43.

In the presently preferred embodiment, the data resident in the lookup table of the controlling computer 61 the detection of which might, optionally, modify the appropriate operating range, include any of a number of ambient sensors. These optional data might include:

- an ambient thermometer to measure an ambient temperature of an ambient environment wherein the pump resides and in response to the ambient temperature to generate an ambient temperature signal sent to the controlling computer;

- a hopper thermometer to measure a concrete mix temperature for concrete in the hopper and in response to the concrete mix temperature to generate an concrete temperature signal sent to the controlling computer; or
- a hygrometer to measure the humidity of the ambient environment and in response to the humidity temperature to generate an ambient humidity signal sent to the controlling computer, and a level sensor to measure a volume of concrete in the hopper and in response to the volume of concrete in the hopper to generate a concrete volume signal sent to the controlling computer.

In devices wherein one or a number of these ambient sensors are employed, the operating range is actually a function of the data received from the applicable ambient sensor or sensor. For example, a slump measured at 5°C is quite distinct from a slump measured at 25°C. The appropriate operating range is modified in accord with the ambient temperature or the concrete 21 mix temperature. In an instance of pumping concrete at the cooler temperature, pumping requires a lesser slump to be workable than does pumping at the greater temperature. The most pronounced effects of hot weather on concrete are increased setting rates and rapid water loss. Higher temperatures can cause a lower measured slump, and consequently a decrease in workability.

Under normal weather, the key concerns in curing will be the maintenance of a moist environment around the concrete. Temperature variations are not a major problem, provided the concrete temperature is maintained above 5 degrees Celsius. Under hot weather conditions, the high temperatures are likely to result in excessive moisture loss. Curing in cold weather will be different as in this case the biggest concern will be the maintaining of an adequate and conducive temperature for hydration. For massive formed concrete members, the heat generated by the concrete during hydration will be adequate to provide a satisfactory curing temperature. The most crucial time for concrete strength gains is that immediately following placement. For this reason, optionally selectively retrieving data reflecting the then-current conditions such as ambient temperature and humidity at the time of pumping, that data along with the measured slump,

Refining the operating range as a function of these optional variable assures an optimal filling of the forms resulting in the best concrete pour possible under those conditions. In field conditions, heat and wind can dry out the moisture from the placed mixture. For that reason, the inventive system and method not only assures the avoiding hazards to operators based upon entrapping compressed air in pump cylinders and but also assuring optimal strength in the resulting concrete structures.

In performing the described actions, the controlling computer 61, may, optionally, be selected to be a programmable logic controller. Programmable logic controllers (PLC) 61 (and will be referred to herein interchangeably with controlling computer 61) are industrial computers that contain hardware and software used to perform control functions in response to sensor signals received from various sensors. More specifically, a PLC 61 enables automation of industrial electromechanical processes having multiple arrangements of digital and analog inputs and outputs.

Generally, PLCs 61 are rugged to operate in extended temperature ranges, having immunity to electrical noise, and unaffected by vibration and impact. The PLC 61 comprises two basic sections: a central processing unit (CPU) and an Input/Output (I/O) interface system. The I/O modules are used to bring input signals into the PLC's CPU and output control signals to controlled devices such as motors, valves, sensors and actuators, among others. Additionally, the computer may include a keyboard 67 (this need not be a QWERTY keyboard but may be of a custom abbreviated form to allow specialized control signals to be composed to the PLC 61), a display 63 and an audio modulation system to such as a sound board and appropriate speakers referred to herein as simply as a speaker 65.

The controlling computer's electronic monitoring relies upon the ability to react to the sensed data and thus requires some form of lookup table. While monitoring slump is the simplest embodiment of the controlling computer 61 the invention comprises, as has already been discussed, knowing the temperature of the mix is also extremely useful. Alternately, the volume of concrete 21 in the hopper 20 can be immediately and repeatedly measured using conventional means. Because the inflow of concrete mix can be measured, and the drawn output of the hopper can also be known, a residence time in the hopper can also be calculated. As stated above, the lookup table can be populated with known values depending upon concrete temperature, ambient temperature, humidity of the ambient atmosphere, and volume of concrete in the hopper. A sophisticated series of operating ranges can be generated based upon known values relative to each of these sensed ambient conditions. In such a manner, the optimal pour of concrete can be assured in pumping in each circumstance.

Various further embodiments of the invention might be realized, as well, by installing selected elements on an existing pump 10 and hopper 20 assembly. As can be readily recognized in the review of the invention as described herein the device does encompass many conventional elements that might already be present on a concrete pumping assembly as those that might be purchased from such as Schwing® or from Putzmeister® as nonlimiting examples. Such is advantageous in that the invention can be retrofitted into conventional concrete pumps 10. Some embodiments of the invention can be practiced by use of provided kits that would allow owners to suitably alter their existing commercial-off-the-shelf devices to conform to the above-described invention.

While the preferred embodiment of the invention has been illustrated and described, as noted above, many changes can be made without departing from the spirit and scope of the invention. Accordingly, the scope of the invention is not limited by the disclosure of the preferred embodiment. Instead, the invention should be determined entirely by reference to the claims that follow.

CLAUSES:

1. A method for measuring slump of concrete as the concrete pools in a hopper feeding a concrete pump, the method comprising:

providing an ammeter configured to measure electrical current passing between a power source and electric motor the concrete pump comprises, the electric motor being configured to rotate an agitator shaft the concrete pump further comprises, the agitator shaft including agitator paddles extending radially from the agitator shaft, the ammeter generating an ammeter signal corresponding to the slump of the concrete in the hopper in response to the electrical current as measured at the ammeter;
providing a controlling computer, the controlling computer including:

a central processing unit (CPU);
an input/output system, the input/output system allowing the CPU:

to respond to the pressure gauge signal; and
to generate an alert signal in response to instructions from the CPU

a memory in which an operating range may reside to be retrieved by the CPU;

defining the operating range extending from a lower ammeter signal value corresponding to a lower limit of slump value to an upper ammeter signal value corresponding to an upper limit of slump value; and
as the concrete pump draws concrete from the hopper, comparing, by means of the controlling computer, the ammeter signal to the defined operating range to determine whether the ammeter signal falls outside of the operating range and when the signal falls outside of operating range, to generate the alert signal.

2. The method of Clause 1, wherein the operating range is stored in a lookup table, the lookup table being resident in a memory in the controlling computer.

3. The method of any preceding Clause, wherein the method further comprises:

providing an ambient sensor selected from an ambient sensor group, the ambient sensor group constituting an ambient thermometer to measure an ambient temperature of an ambient environment wherein the pump resides and in response to the ambient temperature to generate an ambient temperature signal sent to the controlling computer, a hopper thermometer to measure a concrete mix temperature for concrete in the hopper and in response to the concrete mix temperature to generate an concrete temperature signal sent to the controlling computer, a hygrometer to measure the humidity of the ambient environment and in response to the humidity temperature to generate an ambient humidity signal sent to the controlling computer, and a level sensor to measure a volume of concrete in the hopper and in response to the volume of concrete in the hopper to generate a concrete volume signal sent to the controlling computer; and

wherein defining the operating range included defining the operating range as a function of each signal received from any of the ambient sensor group.

4. The method of any preceding Clause further comprising:

detecting the alert signal in the event the controlling computer generates the alert signal; and

stopping operation of the concrete pump in response to the alert signal in the event the alert signal is detected.

5. The method of any preceding Clause further comprising:

detecting the alert signal in the event the controlling computer generates the alert signal; and

generating a warning, the warning selected from a warning group consisting of an audible warning on a speaker, a graphic warning generated on a display, and a radio signal transmitted by means of a Bluetooth® modem cooperating with a Bluetooth® antenna.

6. The method of any preceding Clause, wherein

7. A method for measuring slump of concrete as the concrete pools in a hopper feeding a concrete pump, the method comprising:

providing a pressure gauge configured to measure hydraulic pressure in a hydraulic drive hose passing between a hydraulic pump and a hydraulic motor the concrete pump comprises, the hydraulic motor being configured to rotate an agitator shaft the concrete pump further comprises, the agitator shaft including agitator paddles extending radially from the agitator shaft, the pressure gauge generating a pressure gauge signal corresponding to the slump of the concrete in the hopper in response to the pressure gauge as measured at the hydraulic drive hose;
providing a controlling computer, the controlling computer including:

a central processing unit (CPU);
an input/output system, the input/output system allowing the CPU:

to respond to the pressure gauge signal; and
to generate an alert signal in response to instructions from the CPU

a memory in which an operating range may reside to be retrieved by the CPU;

defining the operating range extending from a pressure gauge signal value corresponding to a lower limit of slump value to a pressure gauge signal value corresponding to an upper limit of slump value; and
as the concrete pump draws concrete from the hopper, comparing, by means of the controlling computer, the pressure gauge signal to the defined operating range to determine whether the pressure gauge signal falls outside of the operating range and when the signal falls outside of operating range, to generate the alert signal.

8. The method of Clause 7, wherein the operating range is stored in a lookup table, the lookup table being resident in a memory in the controlling computer.

9. The method of Clause 7 or 8, wherein the method further comprises:

providing an ambient sensor selected from an ambient sensor group, the ambient sensor group constituting an ambient thermometer to measure an ambient temperature of an ambient environment wherein the pump resides and in response to the ambient temperature to generate an ambient temperature signal sent to the controlling computer, a hopper thermometer to measure a concrete mix temperature for concrete in the hopper and in response to the concrete mix temperature to generate an concrete temperature signal sent to the controlling computer, a hygrometer to measure the humidity of the ambient environment and in response to the humidity temperature to generate an ambient humidity signal sent to the controlling computer, and a level sensor to measure a volume of concrete in the hopper and in response to the volume of concrete in the hopper to generate a concrete volume signal sent to the controlling computer; and

wherein defining the operating range included defining the operating range as a function of each signal received from any of the ambient sensor group.

10. The method of any of Clauses 7 to 9 further comprising:

detecting the alert signal in the event the controlling computer generates the alert signal; and

stopping operation of the concrete pump in response to the alert signal in the event the alert signal is detected.

11. The method of any of Clauses 7 to 10 further comprising:

detecting the alert signal in the event the controlling computer generates the alert signal; and generating a warning, the warning selected from a warning group consisting of an audible warning on a speaker, a graphic warning generated on a display, and a radio signal transmitted by means of a Bluetooth® modem cooperating with a Bluetooth® antenna.

12. The method of any of Clauses 7 to 11, wherein the controlling computer generates a graphic representation of a slump value corresponding to the pressure gauge signal corresponding to the slump of the concrete in the hopper on a display connected to the controlling computer.

13. A device for measuring slump of concrete in a concrete pump hopper feeding a concrete pump, the concrete pump including an agitator shaft the concrete pump further comprises, the agitator shaft including agitator paddles extending radially from the agitator shaft, the device comprising:

a slump sensor selected from a slump sensor group, the slump sensor to generate a slump sensor signal, the slump sensor group consisting of:

an ammeter to measure current from a power source feeding an electric motor being configured to rotate the agitator shaft, the ammeter generating the slump sensor signal corresponding to the slump of the concrete in the hopper in response to the electrical current as measured at the ammeter; and
a pressure gauge configured to measure hydraulic pressure in a hydraulic drive hose passing between a hydraulic pump and a hydraulic motor the concrete pump comprises, the hydraulic motor being configured to rotate the agitator shaft, the pressure gauge generating the slump sensor signal corresponding to the slump of the concrete in the hopper in response to the hydraulic pressure as measured at the hydraulic drive hose; and

a controlling computer, the controlling computer including:

a central processing unit (CPU);
a memory in which an operating range may reside to be retrieved by the CPU, the operating range being defined as extending from a lower slump signal value corresponding to a lower limit of slump value to an upper slump signal value corresponding to an upper limit of slump value;
an input/output system, the input/output system allowing the CPU:

to respond to the slump sensor signal; and
to generate an alert signal in response to instructions from the CPU in response to a slump sensor signal received at the CPU in that the slump sensor signal falls outside of the operating range.

14. The device of Clause 13, wherein the operating range is stored in a lookup table, the lookup table being resident in the memory.

15. The device of Clause 13 or 14, further comprising:

an ambient sensor selected from an ambient sensor group, the ambient sensor group constituting an ambient thermometer to measure an ambient temperature of an ambient environment wherein the pump resides and in response to the ambient temperature to generate an ambient temperature signal sent to the controlling computer, a hopper thermometer to measure a concrete mix temperature for concrete in the hopper and in response to the concrete mix temperature to generate an concrete temperature signal sent to the controlling computer, a hygrometer to measure the humidity of the ambient environment and in response to the humidity temperature to generate an ambient humidity signal sent to the controlling computer, and a level sensor to measure a volume of concrete in the hopper and in response to the volume of concrete in the hopper to generate a concrete volume signal sent to the controlling computer; and

wherein defining the operating range included defining the operating range as a function of each signal received from any of the ambient sensor group.

16. The device of any of Clauses 13 to 15 further comprising a warning device to generate a warning, the warning selected from a warning group consisting of an audible warning on a speaker, a graphic warning generated on a display, and a radio signal transmitted by means of a Bluetooth® modem cooperating with a Bluetooth® antenna.

17. The device of any of Clauses 13 to 16, further comprising a concrete pump controller, the concrete pump controller configured to interrupt the concrete pump in pumping, the interrupting being in response to the alert signal when the alert signal is generated in the input/output system.

18. The device of any of Clauses 13 to 17, further comprises a display operatively connected to the input/output system, wherein the controlling computer generates a graphic representation of a slump value corresponding to the slump sensor signal corresponding to the slump of the concrete in the hopper on the display.

19. The device of any of Clauses 13 to 18, wherein the input/output system further comprises a keyboard for interacting with the CPU.

20. The device of any of Clauses 13 to 19, wherein the controlling computer includes a programmable logic controller.

**Claims**

1. A method for measuring slump of concrete as the concrete pools in a hopper feeding a concrete pump, the method comprising:

   providing an ammeter configured to measure electrical current passing between a power source and electric motor the concrete pump comprises, the electric motor being configured to rotate an agitator shaft the concrete pump further comprises, the agitator shaft including agitator paddles extending radially from the agitator shaft, the ammeter generating an ammeter signal corresponding to the slump of the concrete in the hopper in response to the electrical current as measured at the ammeter;
   providing a controlling computer, the controlling computer including:

      a central processing unit (CPU);
      an input/output system, the input/output system allowing the CPU:

         to respond to the pressure gauge signal; and
         to generate an alert signal in response to instructions from the CPU

      a memory in which an operating range may reside to be retrieved by the CPU;

   defining the operating range extending from a lower ammeter signal value corresponding to a lower limit of slump value to an upper ammeter signal value corresponding to an upper limit of slump value; and
   as the concrete pump draws concrete from the hopper, comparing, by means of the controlling computer, the ammeter signal to the defined operating range to determine whether the ammeter signal falls outside of the operating range and when the signal falls outside of operating range, to generate the alert signal.

2. A method for measuring slump of concrete as the concrete pools in a hopper feeding a concrete pump, the method comprising:

   providing a pressure gauge configured to measure hydraulic pressure in a hydraulic drive hose passing between a hydraulic pump and a hydraulic motor the concrete pump comprises, the hydraulic motor being configured to rotate an agitator shaft the concrete pump further comprises, the agitator shaft including agitator paddles extending radially from the agitator shaft, the pressure gauge generating a pressure gauge signal corresponding to the slump of the concrete in the hopper in response to the pressure gauge as measured at the hydraulic drive hose;
   providing a controlling computer, the controlling computer including:

a central processing unit (CPU);
an input/output system, the input/output system allowing the CPU:

to respond to the pressure gauge signal; and
to generate an alert signal in response to instructions from the CPU

a memory in which an operating range may reside to be retrieved by the CPU;

defining the operating range extending from a pressure gauge signal value corresponding to a lower limit of slump value to a pressure gauge signal value corresponding to an upper limit of slump value; and
as the concrete pump draws concrete from the hopper, comparing, by means of the controlling computer, the pressure gauge signal to the defined operating range to determine whether the pressure gauge signal falls outside of the operating range and when the signal falls outside of operating range, to generate the alert signal.

3. The method of any preceding Claim, wherein the operating range is stored in a lookup table, the lookup table being resident in a memory in the controlling computer.

4. The method of any preceding Claim, wherein the method further comprises:

providing an ambient sensor selected from an ambient sensor group, the ambient sensor group constituting an ambient thermometer to measure an ambient temperature of an ambient environment wherein the pump resides and in response to the ambient temperature to generate an ambient temperature signal sent to the controlling computer, a hopper thermometer to measure a concrete mix temperature for concrete in the hopper and in response to the concrete mix temperature to generate an concrete temperature signal sent to the controlling computer, a hygrometer to measure the humidity of the ambient environment and in response to the humidity temperature to generate an ambient humidity signal sent to the controlling computer, and a level sensor to measure a volume of concrete in the hopper and in response to the volume of concrete in the hopper to generate a concrete volume signal sent to the controlling computer; and
wherein defining the operating range included defining the operating range as a function of each signal received from any of the ambient sensor group.

5. The method of any preceding Claim further comprising:

detecting the alert signal in the event the controlling computer generates the alert signal; and stopping operation of the concrete pump in response to the alert signal in the event the alert signal is detected.

6. The method of any preceding Claim further comprising:

detecting the alert signal in the event the controlling computer generates the alert signal; and generating a warning, the warning selected from a warning group consisting of an audible warning on a speaker, a graphic warning generated on a display, and a radio signal transmitted by means of a Bluetooth® modem cooperating with a Bluetooth® antenna.

7. The method of any preceding Claim, wherein the controlling computer generates a graphic representation of a slump value corresponding to the pressure gauge signal corresponding to the slump of the concrete in the hopper on a display connected to the controlling computer.

8. A device for measuring slump of concrete in a concrete pump hopper feeding a concrete pump, the concrete pump including an agitator shaft the concrete pump further comprises, the agitator shaft including agitator paddles extending radially from the agitator shaft, the device comprising:

a slump sensor selected from a slump sensor group, the slump sensor to generate a slump sensor signal, the slump sensor group consisting of:

an ammeter to measure current from a power source feeding an electric motor being configured to rotate the agitator shaft, the ammeter generating the slump sensor signal corresponding to the slump of the concrete in the hopper in response to the electrical current as measured at the ammeter; and
a pressure gauge configured to measure hydraulic pressure in a hydraulic drive hose passing between a hydraulic pump and a hydraulic motor the concrete pump comprises, the hydraulic motor being configured to rotate the agitator shaft, the pressure gauge generating the slump sensor signal corresponding to the slump of the concrete in the hopper in response to the hydraulic pressure as measured at the hydraulic drive hose; and

a controlling computer, the controlling computer including:

a central processing unit (CPU);
a memory in which an operating range may reside to be retrieved by the CPU, the operating range being defined as extending from a lower slump signal value corresponding to a lower limit of slump value to an upper slump signal value corresponding to an upper limit of slump value;
an input/output system, the input/output system allowing the CPU:

to respond to the slump sensor signal; and
to generate an alert signal in response to instructions from the CPU in response to a slump sensor signal received at the CPU in that the slump sensor signal falls outside of the operating range.

9. The device of Claim 8, wherein the operating range is stored in a lookup table, the lookup table being resident in the memory.

10. The device of Claim 8 or 9, further comprising:

an ambient sensor selected from an ambient sensor group, the ambient sensor group constituting an ambient thermometer to measure an ambient temperature of an ambient environment wherein the pump resides and in response to the ambient temperature to generate an ambient temperature signal sent to the controlling computer, a hopper thermometer to measure a concrete mix temperature for concrete in the hopper and in response to the concrete mix temperature to generate an concrete temperature signal sent to the controlling computer, a hygrometer to measure the humidity of the ambient environment and in response to the humidity temperature to generate an ambient humidity signal sent to the controlling computer, and a level sensor to measure a volume of concrete in the hopper and in response to the volume of concrete in the hopper to generate a concrete volume signal sent to the controlling computer; and
wherein defining the operating range included defining the operating range as a function of each signal received from any of the ambient sensor group.

11. The device of any of Claims 8 to 10 further comprising a warning device to generate a warning, the warning selected from a warning group consisting of an audible warning on a speaker, a graphic warning generated on a display, and a radio signal transmitted by means of a Bluetooth® modem cooperating with a Bluetooth® antenna.

12. The device of any of Claims 8 to 11, further comprising a concrete pump controller, the concrete pump controller configured to interrupt the concrete pump in pumping, the interrupting being in response to the alert signal when the alert signal is generated in the input/output system.

13. The device of any of Claims 8 to 12, further comprises a display operatively connected to the input/output system, wherein the controlling computer generates a graphic representation of a slump value corresponding to the slump sensor signal corresponding to the slump of the concrete in the hopper on the display.

14. The device of any of Claims 8 to 13, wherein the input/output system further comprises a keyboard for interacting with the CPU.

15. The device of any of Claims 8 to 14, wherein the controlling computer includes a programmable logic controller.

**FIG.1**

**FIG.2**

EP 4 450 245 A1

Europäisches
Patentamt

European
Patent Office

Office européen
des brevets

# EUROPEAN SEARCH REPORT

Application Number

EP 23 16 8822

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2013 174619 A (KAJIMA CORP) 5 September 2013 (2013-09-05) | 2,3,6-9, 11,13-15 | INV. B28C7/02 |
| A | * paragraph [0020] – paragraph [0045]; figures * | 1,4,5, 10,12 | B28C7/16 E04G21/02 F04B15/02 G01N33/38 |
| X | JP 2010 249742 A (KAJIMA CORP) 4 November 2010 (2010-11-04) | 2,3,6-9, 11,13-15 | |
| A | * paragraph [0014] – paragraph [0036]; figures * | 1,4,5, 10,12 | |
| A | US 6 227 039 B1 (TE ENI MOSHE [IL]) 8 May 2001 (2001-05-08) * column 13, line 40 – column 14, line 11; figures 9A,9B * | 1,8-15 | |

TECHNICAL FIELDS SEARCHED (IPC)

B28C
F04B
E04G
G01N

The present search report has been drawn up for all claims

| Place of search | Date of completion of the search | Examiner |
|---|---|---|
| The Hague | 6 September 2023 | Orij, Jack |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 8822

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2023

| Patent document cited in search report | | Publication date | Patent family member(s) | | Publication date |
|---|---|---|---|---|---|
| JP 2013174619 | A | 05-09-2013 | JP | 5643384 B2 | 17-12-2014 |
| | | | JP | 2013174619 A | 05-09-2013 |
| JP 2010249742 | A | 04-11-2010 | JP | 5283555 B2 | 04-09-2013 |
| | | | JP | 2010249742 A | 04-11-2010 |
| US 6227039 | B1 | 08-05-2001 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 63252002 A, Joseph Sostaric